# EUROPÄISCHE PATENTSCHRIFT

(11) **EP 4 304 498 B1**
(45) Veröffentlichungstag und Bekanntmachung des Hinweises auf die Patenterteilung: **17.04.2024**
(21) Anmeldenummer: 23714558.6
(22) Anmeldetag: 28.03.2023
(51) Int. Cl.: A61B 17/295, A61B 18/14, A61B 17/28, A61B 17/29

(54) **CHIRURGISCHES INSTRUMENT MIT VERRIEGELUNGSMECHANISMUS**
SURGICAL INSTRUMENT HAVING A LOCKING MECHANISM
INSTRUMENT CHIRURGICAL AYANT UN MÉCANISME DE VERROUILLAGE

(30) Priorität: 04.04.2022 DE 102022108032
(43) Veröffentlichungstag der Anmeldung: 17.01.2024
(73) Patentinhaber: AESCULAP AG, 78532 Tuttlingen (DE)
(72) Erfinder: HAFNER, Nikolaus, 78532 Tuttlingen (DE); CIK, Justin Danny, 78086 Brigachtal (DE)
(74) Vertreter: Winter, Brandl - Partnerschaft mbB
(86) Internationale Anmeldenummer: PCT/EP2023/058020
(87) Internationale Veröffentlichungsnummer: WO 2023/194160

(56) Entgegenhaltungen:
- EP-A1- 3 235 445
- EP-B1- 3 400 893
- DE-A1-102011 001 372
- US-A1- 2006 089 670
- US-A1- 2014 031 821

## Beschreibung

Die vorliegende Offenbarung betrifft ein chirurgisches Instrument zum Klemmen und Trennen von Gewebe (Patientengewebe), mit einer ersten Instrumentenbranche und einer an der ersten Instrumentenbranche verschwenkbar angelenkten bzw. gelagerten zweiten Instrumentenbranche, welche jeweils eine distale Klemmbacke und ein proximales Griffelement ausbilden, wobei die zweite Instrumentenbranche ein Gewebetrennelement zum Durchtrennen von zwischen den Klemmbacken gehaltenem Patientengewebe und einen Antriebsmechanismus zum Antreiben des Gewebetrennelements aufweist, und wobei ein Riegelelement zum Verriegeln oder Freigeben des Antriebsmechanismus bereitgestellt ist.

### Stand der Technik

Chirurgische Versiegelungs- und Schneidinstrumente (Seal & Cut Instrumente) werden verwendet, um bei einer Operation Patientengewebe zu greifen, zu versiegeln und zu durchtrennen bzw. zu schneiden. Diese weisen in der Regel eine distale Instrumentenspitze auf, an welcher Klemmbacken zum Greifen und Klemmen des Patientengewebes bereitgestellt ist. Zudem haben solche Instrumente ein Trenn- oder Schneidelement, welches beispielsweise eine Klinge oder eine Trennkante aufweist, welche das Patientengewebe durchtrennt. Handgehaltene Instrumente haben darüber hinaus einen Griff, welchen der Anwender halten kann und an welchem verschiedene Betätigungselemente zum Betätigen einer Bewegung der Klemmbacken und/ oder zum Betätigen des Trenn- oder Schneidelements bereitgestellt sind. Um zu vermeiden, dass das Trenn- oder Schneidelement ungewollt betätigt wird, ist dieses meist ver- und entriegelbar.

Beispielsweise ist aus EP 3 400 893 B1 eine entsprechende elektrochirurgische Zange bekannt, mit proximalen Griffbranchen, distalen Klemmbacken und einer Klinge, die zwischen einer zurückgezogenen und einer ausgefahrenen Position verschiebbar ist. In einer der Griffbranchen ist eine Klingenverriegelung bereitgestellt, die eine distale Verschiebung der Klinge verhindert und bei einer Annäherung der Klemmbacken aus der verriegelten Position in eine entriegelte Position bewegbar ist, um eine distale Verschiebung der Klinge zu ermöglichen. Die Klingenverriegelung hat einen Haken, welcher wahlweise mit einem Klingenantriebsgestänge in Eingriff bringbar ist, um dieses zu verriegeln. Ein mit dem Haken verbundener Finger ragt in Richtung der anderen Griffbranche vor. Werden die Griffbranchen aufeinander zu bewegt, drückt die andere Griffbranche gegen den Finger, wodurch der Haken von dem Klingenantriebsgestänge gelöst wird und dieses somit entriegelt.

Ferner, US 2014/031821 A1 offenbart ein chirurgisches Instrument, welches zwei Schaftelemente, die jeweils ein Backenelement aufweisen, eine Abzugsanordnung, die zwischen einer unbetätigten Position und einer betätigter Position bewegbar ist, um eine Klinge wahlweise zwischen einer zurückgezogenen Position und mindestens einer ausgefahrenen Position zu verschieben.

DE 10 2011 001372 A1 offenbart ein chirurgisches Instrument mit mindestens zwei relativ zueinander bewegbaren Werkzeugelementen, welche jeweils eine HF-Elektrode umfassen, die in einer Annäherungsstellung der Werkzeugelemente einen minimalen Abstand voneinander definieren, einander gegenüberliegen und aufeinander zu weisen, um infolge einer Beaufschlagung mit einem HF-Strom zwischen den Werkzeugelementen gehaltenes Körpergewebe zu verbinden, und mit einem Schneidelement zum Durchtrennen und/oder Abtrennen von Körpergewebe.

EP 3 235 445 A1 offenbar ein chirurgisches Klammersystem mit einer Sperre. Die Sperre ist so konfiguriert, dass sie verhindert, dass das Heftsystem eine Heftauslösefunktion ausführt, wenn ein nicht verbrauchtes Heftklammermagazin nicht vollständig im Heftsystem sitzt.

Problematisch am Stand der Technik ist, dass an einer Stelle, an welcher der Finger aus dem Instrumentengehäuse herausragt, eine Öffnung vorgesehen sein muss. Dadurch können Partikel, die durch mechanische Reibung im Instrument entstehen durch die Öffnung in die Wunde bzw. die Operationsstelle am Patienten fallen und dort als ungewollter Fremdstoff / Fremdkörper vorliegen, wodurch der Patient gefährdet wird. D.h. eine Kontaminationsgefahr ist hoch. Darüber hinaus können Flüssigkeiten (NaCl, Blut, etc.) ungewollt in das Instrument eindringen und dort die Mechanik / Elektronik behindern oder beschädigen. Um diese Probleme zu reduzieren, muss die Stelle abgedichtet bzw. isoliert werden, wodurch das Instrument aufwändiger und teurer wird. Zudem kann der Stift abbrechen, insbesondere, wenn er aus Kunststoff gefertigt ist. Darüber hinaus ist der Verriegelungsmechanismus relativ komplex. Seine Funktion kann insbesondere durch Alterungseinflüsse auf Kunststoffteile beeinträchtigt werden. Weiterhin ist es nachteilig, dass die Messerverriegelung einfach und ohne jegliche Hilfsmittel vom Anwender überbrückbar ist, indem er händisch gegen den Stift drückt oder daran hängen bleibt. Dadurch ist eine Verletzungsgefahr für den Anwender sowie ein Risiko, Patientengewebe ungewollt zu durchtrennen, erhöht. In einem Fall, in welchem eine Klingenbetätigung vorbelastet wird, bevor die Klinge entriegelt ist, kann es ferner dazu kommen, dass die Klinge schlagartig vorschnappt, wenn sie schließlich entriegelt wird. Dies kann ebenfalls ein ungewolltes oder unkontrolliertes Schneiden zur Folge haben und stellt somit ein hohes Sicherheitsrisiko dar.

### Zusammenfassung der Erfindung

Die der vorliegenden Offenbarung zugrundeliegende Aufgabe besteht darin, Nachteile des Stands der Technik zu verbessern oder zu beseitigen. Insbesondere soll ein chirurgisches Instrument zum Klemmen und Trennen von Patientengewebe mit bereitgestellt werden, welches besonders robust und sicher anwendbar ist.

Die der Offenbarung zugrundeliegende Aufgabe wird durch ein chirurgisches Instrument mit den Merkmalen des Anspruchs 1 gelöst. Vorteilhafte Ausgestaltungen sind Gegenstand der Unteransprüche und werden später genauer beschrieben.

Genauer ausgedrückt, wird die der Offenbarung zugrundeliegende Aufgabe gelöst durch ein chirurgisches Instrument zum Klemmen und Trennen von Patientengewebe, mit einer ersten Instrumentenbranche und einer an der ersten Instrumentenbranche verschwenkbar angelenkten zweiten Instrumentenbranche, welche jeweils eine distale Klemmbacke und ein proximales Griffelement ausbilden. Die zweite Instrumentenbranche hat ein Gewebetrennelement zum Durchtrennen von zwischen den Klemmbacken gehaltenem Patientengewebe und einen Antriebsmechanismus zum Antreiben des Gewebetrennelements. Ferner lagert die zweite Instrumentenbranche ein Riegelelement derart, dass es zwischen einer Verriegelungsstellung, in welcher es in den Antriebsmechanismus blockierend eingreift, und einer Offenstellung, in welcher der Antriebsmechanismus durch das Riegelelement unblockiert ist, beweglich ist. Das Riegelelement hat einen magnetischen Riegelabschnitt. Die erste Instrumentenbranche hat an einem dem Riegelelement gegenüberliegenden Bereich einen magnetischen Betätigungsabschnitt zur Betätigung des Riegelelements. Der magnetische Riegelabschnitt und/ oder der magnetische Betätigungsabschnitt hat/ haben einen Magneten, um bei einer Annäherung der Instrumentenbranchen zueinander das Riegelelement durch eine magnetische Anziehung oder Abstoßung zwischen dem magnetischen Riegelabschnitt und dem ersten magnetischen Betätigungsabschnitt in die Offenstellung zu bewegen und eine Betätigung des Gewebetrennelements freizugeben.

In anderen Worten ausgedrückt, ist ein gattungsgemäßes chirurgisches Instrument bereitgestellt, mit einem Riegelelement zum Verriegeln des Trenn- und Schneidelements, welches in blockierenden Eingriff mit dem Antriebsmechanismus bringbar ist. Im blockierenden Eingriff, d.h. in einer Verriegelungsstellung, ist der Antriebsmechanismus blockiert und kann sich nicht oder nur geringfügig bewegen. Somit kann das Gewebetrennelement nicht gewebetrennend betätigt werden. Ist das Riegelelement mit dem Antriebsmechanismus außer Eingriff, d.h. in einer Offenstellung, kann der Antriebsmechanismus und dadurch das Gewebetrennelement durch den Anwender wahlweise betätigt werden. Das Riegelelement ist zumindest teilweise magnetisch und kann durch eine gezielte Annäherung oder Wegnahme eines magnetischen Betätigungsabschnitts betätigt werden. Das Riegelelement und/ oder der Betätigungsabschnitt weist einen Magneten, insbesondere einen Permanentmagneten auf. Das Riegelelement ist insbesondere quer zur Erstreckungsrichtung der Instrumentenbranchen beweglich gelagert.

Als magnetisch ist ein Materialabschnitt oder Bauteil anzusehen, welcher selbst ein Magnet ist (ein Magnetfeld erzeugt), wie z.B. ein Neodymmagnet, oder welches durch ein externes Magnetfeld magnetisch anziehbar ist, aber nicht unbedingt selbst einen Magneten aufweist. Dies kann beispielsweise ein ferromagnetisches Metall wie Stahl oder auch ein Permanentmagnet sein. Im Fall einer Ausführung basierend auf einer Anziehungskraft kann vorteilhafter Weise (wie nachstehend genauer beschrieben) ein schnelles Umschnappen des Riegelelements zwischen der Verriegelungsstellung der Offenstellung erreicht werden. Bei einer geeigneten Auslegung der entsprechenden Parameter (z.B. Position, Form, Größe, Material der magnetischen Elemente) kann im Fall einer Ausführung basierend auf einer Abstoßungskraft vorteilhafter Weise (wie nachstehend genauer beschrieben) ein stoßarmes, graduelles Wechseln des Riegelelements zwischen der Verriegelungsstellung der Offenstellung erreicht werden. Alternativ kann die Auslegung auch so gewählt werden, dass ein ruckartiges Wechseln erreicht wird.

Dies hat den besonderen Vorteil, dass eine Anzahl notwendiger Öffnungen im Instrumentengehäuse minimiert wird. Dadurch wird auch eine Kontaminationsgefahr reduziert. Sämtliche Teile des Antriebsmechanismus können zudem geschützt im Instrumentengehäuse angeordnet sein, sodass ein Beschädigungsrisiko minimiert ist. In anderen Worten kann die Klingenverrieglung vollständig gekapselt bzw. vollständig im Gehäuse verbaut sein. Es gibt also keine aus dem Gehäuse herausragenden Komponenten und ist keine Abdichtung, Isolierung etc. nötig. Ferner kann das Instrument einfacher und kostengünstiger ausgebildet sein. Ein ungewolltes Entriegeln des Antriebsmechanismus ist zudem nahezu ausgeschlossen, da hierfür ein gesonderter Magnet notwendig ist. Somit ist ein Verletzungsrisiko durch eine ungewollte oder unkontrollierte Betätigung des Gewebetrennelements minimiert. D.h. die Klingenverriegelung kann nicht ohne Hilfsmittel manipuliert oder überlistet werden.

Insbesondere ist es von Vorteil, wenn das Riegelelement in einer Richtung durch das Betätigungselement (oder durch das Rückstellelement) angezogen wird. Da eine magnetische Anziehungskraft selbstverstärkend ist, kann dadurch erreicht werden, dass das Riegelelement aus der Verriegelungsstellung in die Offenstellung umschnappt, d.h. sehr schnell zur Verriegelungsstellung wechseln kann. Dadurch kann beispielsweise eine halb entriegelte Stellung des Riegelelements vermieden werden, sodass das Risiko einer Situation minimierbar ist, in welcher das Gewebetrennelement schlagartig eine Trennbewegung durchführt (z.B. vorschnappt), wenn ein Schalter zur Betätigung des Gewebetrennelement gerade betätigt wird und unter einer solchen Vorbelastung ein halb entriegeltes Riegelelement verrutscht. In anderen Worten kann das Riegelelement nicht in die freigegebene Stellung wechseln, wenn die Klingenbetätigung vorgespannt wird.

Das Gewebetrennelement kann beispielsweise eine Klinge oder eine Kante aufweisen, welche an das durch die Klemmbacken gehaltene Patientengewebe angedrückt/ bewegt wird und dieses durchtrennt. Zum Gewebetrennen kann das Gewebetrennelement in Richtung distal vorschiebbar in der zweiten Instrumentenbranche gelagert sein. Alternativ oder zusätzlich kann das Gewebetrennelement an einer der Klemmbacken schwenkbar gelagert sein. Der Antriebsmechanismus kann das Gewebetrennelement mit einem Betätigungsschalter verbinden, insbesondere auf (rein) mechanische Weise, welcher durch den Anwender betätigbar ist. Der Antriebmechanismus kann z.B. ein Gestänge und/ oder ein Zahnradgetriebe aufweisen. Der Antriebsmechanismus hat also bewegte Bauteile. Die vorliegende Offenbarung macht sich das zunutze und stellt das Riegelelement derart bereit, dass es die Bewegung von einem oder mehreren der bewegten Bauteile wahlweise blockiert.

Die beiden Instrumentenbranchen können an insbesondere an einem Drehgelenk zwischen den Klemmbacken und den Griffelementen schwenkbar aneinander angelenkt sein. Insbesondere werden Abschnitte der Instrumentenbranche distal vom Drehgelenk als Klemmbranche bezeichnet und Abschnitte proximal vom Gelenk als Griffelement bezeichnet. Das Riegelelement und der magnetische Betätigungsabschnitt sind bevorzugt an den Griffelementen bereitgestellt. Alternativ ist es auch denkbar, das Griffelement und den magnetischen Betätigungsabschnitt an den Klemmbacken anzuordnen.

Bevorzugt hat die zweite Instrumentenbranche ein Rückstellelement, welches eine Rückstellkraft an das Riegelelement anlegt, die einer Anziehungskraft oder Abstoßungskraft zwischen dem magnetischen Riegelabschnitt und dem magnetischen Betätigungsabschnitt entgegenwirkt, um eine in Richtung der Verriegelungsstellung wirkende Rückstellkraft auf das Riegelelement auszuüben. Weiter bevorzugt hat das Rückstellelement eine Feder oder ein magnetisches Element.

In anderen Worten ausgedrückt, ist ein Rückstellelement bereitgestellt, welches das Riegelelement in die Verriegelungsposition vorspannt. Dadurch wird ermöglicht, den Riegel zuverlässig wieder in die Verriegelungsstellung zurückzustellen, wenn eine Kraft, welche durch den Betätigungsabschnitt auf das Riegelelement ausgeübt wird, nachlässt. Somit kann eine Position des Riegels zwischen der Offen- und der Verriegelungsstellung kontrolliert eingestellt werden.

Insbesondere ist es von Vorteil, wenn der magnetische Betätigungsabschnitt, der magnetische Riegelabschnitt und das Rückstellelement derart relativ zueinander ausgelegt und positioniert sind, dass das Riegelelement dann in die Offenstellung wechselt, wenn zwischen den Klemmbacken ein vorbestimmter Mindestdruck (eine vorbestimmte Flächenpressung) anliegt. Anders ausgedrückt, kann die Auslegung derart erfolgen, dass die Klingenverriegelung (das Riegelelement) erst ab einem definierten Druck im Maulteil (z.B. einem Mindestdruck von 0,1 N/mm^2 bis 0,5 N/mm^2) entriegelt, also die Klinge freigibt.

Das Riegelelement kann ein Magnet sein (d.h. ein beweglicher Schieber, in dem sich ein (Sperr-) Magnet befindet oder der aus einem (Sperr-) Magnet besteht) und das Rückstellelement und der Betätigungsabschnitt können magnetlos (d.h. ohne einen eigenen Magneten) ausgebildet sein. In diesem Fall wird nur ein Magnet benötigt, was besonders kostengünstig ist. Das Riegelelement kann optional eine Schutzhülle um den ggf. darin aufgenommenen Magneten oder magnetischen Abschnitt bilden (umspritzt/ vollständig dadurch gekapselt sein) oder stoffschlüssig, kraftschlüssig oder formschlüssig damit gekoppelt sein. Alternativ kann das Riegelelement magnetlos sein und kann der Betätigungsabschnitt und ggf. das Rückstellelement einen Magneten oder mehrere Magneten aufweisen. Dies ist vorteilhaft, da das Riegelelement sich zwischen zwei Positionen hin- und herbewegt und dabei in jeweiligen Endpositionen anschlagen kann, wodurch ein Permanentmagnet, der typischerweise spröde Materialien aufweist, beschädigt werden könnte.

Vorteilhafter Weise sind der magnetische Betätigungsabschnitt, der magnetische Riegelabschnitt und das Rückstellelement derart relativ zueinander ausgelegt und positioniert, dass das Riegelelement in einer definierten ersten Stellung der Instrumentenbranchen relativ zueinander in der Verriegelungsstellung gehalten wird und in einer definierten zweiten Stellung der Instrumentenbranchen relativ zueinander in der Offenstellung gehalten wird. Insbesondere sind die distalen Klemmbacken in der ersten Stellung geöffnet und befinden sich in der zweiten Stellung in einer vorbestimmten Klemmstellung.

Anders ausgedrückt können das Rückstellelement, insbesondere wenn dies ein magnetisches Element ist, und/ oder der Betätigungsabschnitt, an einer festen Position in der jeweiligen Instrumentenbranche ausgebildet sein. Von Vorteil dabei ist, dass mit einfachen Mitteln genau eingestellt werden kann, wann sich das Riegelelement zwischen der Offenstellung der Verriegelungsstellung bewegt. In anderen Worten ausgedrückt, kann durch ein Abstimmen der einzelnen Abstände und/ oder der Positionen und/ oder Dimensionen der magnetischen Abschnitte oder Elemente (d.h. des Riegelelements und/oder des Betätigungsabschnitts und/oder des Rückstellelements) und/ oder einer Stärke des/der darin eingesetzten Magnete/n relativ zueinander ein funktionsfähiges Gleichgewicht erreicht werden. Z.B. kann dadurch erreicht werden, dass das Riegelelement genau dann in die Offenstellung wechselt, wenn eine definierte Flächenpressung zwischen den Klemmbacken anliegt oder unmittelbar bevor sich die Klemmbacken berühren oder genau dann, wenn sie in Kontakt miteinander kommen, oder zu jedem anderen beliebigen Zeitpunkt. Bei der Auslegung und Positionierung des Riegelelements, des Betätigungsabschnitts und ggf. des Rückstellelements wird bevorzugt berücksichtigt, welche anderen magnetischen (z.B. metallischen oder magnetischen) Körpern oder Oberflächen in dem Instrument verbaut sind.

Auf diese Weise kann eine Betätigung des Riegelelements unmittelbar daran gekoppelt werden, wann die Klemmbacken sich in einer zum Trennen des Gewebes geeigneten Stellung befinden. Ferner kann ein Trennen des Gewebes außerhalb dieser Stellung vermieden werden.

Das Instrument gemäß der vorliegenden Offenbarung hat ferner den Vorteil, dass ein Wechsel des Riegelelements in die Offenstellung und/oder in die Verriegelungsstellung plötzlich erfolgt. Dadurch kann das Riegelelement an entsprechenden Endpositionen anschlagen. Dieser Anschlag kann, wie nachstehend genauer beschrieben, entweder gedämpft werden oder genutzt werden, um einem Anwender ein Feedback über das Umschalten des Riegelelements bereitzustellen.

Nach einer vorteilhaften Ausgestaltung ist eine Bewegung des Riegelelements durch einen Dämpferanschlag begrenzt. Am Dämpferanschlag und/oder am Riegelelement auf einer dem Dämpferanschlag zugewandten Seite kann in diesem Fall ein Dämpferabschnitt bereitgestellt sein, um ein Auftreffen des Riegelelements auf den Dämpferanschlag zu dämpfen.

In anderen Worten ausgedrückt, wird eine Bewegung des Riegelelements zumindest in einer Richtung bzw. dessen Endanschlag gedämpft. Dies ist insbesondere von Vorteil, wenn der Endanschlag und/ oder das Riegelelement ein sprödes Material aufweisen, das durch ein Anschlagen des Riegelelements beschädigt werden könnte. Der Dämpferabschnitt kann z.B. ein Elastomer und/oder eine Feder und/oder ein Dämpferfluid aufweisen. Z.B. kann ein Zwischenraum zwischen dem Riegelelement 15 und seiner Führung im Bereich seiner Endanschläge/ Endpositionen luftdicht sein oder ausströmende Luft drosseln, sodass die Luft in diesem Zwischenraum jeweils als Dämpferabschnitt dienen kann. Das Riegelelement kann also neben dem magnetischen Riegelabschnitt einen damit gekoppelten (ggf. dämpfenden) Schieberabschnitt aufweisen.

Alternativ oder zusätzlich kann nach einer weiteren vorteilhaften Ausgestaltung eine Bewegung des Riegelelements durch einen Signalgeberanschlag begrenzt sein. Am Signalgeberanschlag und/ oder am Riegelelement an einer dem Signalgeberanschlag zugewandten Seite kann in diesem Fall ein Signalgeberabschnitt bereitgestellt sein, der beim Auftreffen des Riegelelements auf den Signalgeberanschlag ein taktiles und/oder hörbares Feedback erzeugt oder verstärkt, um dem Anwender zu signalisieren, dass die Verriegelungsstellung und/ oder die Offenstellung erreicht ist.

In anderen Worten ausgedrückt, kann bei der Bewegung des Schiebers / dem Wechsel der Stellungen/ Positionen des Riegelelements ein (taktiles) Feedback bzw. ein Geräusch (ein Klick) erzeugt werden. Ein Signalgeberanschlag ist ein Anschlag des Riegelelements an einer seiner Endpositionen, in welcher beim Anschlagen ein hörbares oder taktiles Signal entsteht, z.B. dadurch, dass zwei harte Oberflächen aufeinandertreffen. Z.B. kann der Signalgeberanschlagabschnitt ein sprödes und/oder hell oder laut klingendes und/oder schwingendes und/ oder vibrierendes Material aufweisen. Dadurch kann das Feedback bzw. das Signal beim Anschlagen des Riegelelements am Signalgeberanschlag erzeugt oder verstärkt werden. Durch das Feedback/ das Signal wird für den Anwender vorteilhafter Weise ersichtlich, dass er eine bestimmte Position der Klemmbacken zueinander erreicht hat, z.B. eine vorbestimmte Flächenpressung zwischen den Klemmbacken vorliegt, die für das Gewebetrennen und ggf. eine Gewebekoagulation benötigt wird. Darüber hinaus erkennt der Anwender dadurch unmittelbar, dass er nun das Gewebetrennelement betätigen kann. Das Signal kann somit als Sicherheitsfeature genutzt werden.

Das chirurgische Instrument kann ferner ein Instrumentengehäuse aufweisen, welches bevorzugt im Bereich des Riegelelements ein Fenster aufweist. Ein hinter dem Fenster liegender Bereich des Riegelelements kann einen sichtbaren Markerabschnitt ausbilden, welcher den Verriegelungszustand und/oder den Öffnungszustand kennzeichnet.

In anderen Worten ausgedrückt hat bevorzugt ein Abschnitt des Riegelelements, welcher im Öffnungszustand hinter dem Fenster liegt (d.h. dadurch am Instrumentenäußeren sichtbar ist), eine erste Kennzeichnung (z.B. rot sein) und hat ein weiterer Abschnitt des Riegelelements, welcher im Verriegelungszustand hinter dem Fenster liegt, eine zweite Kennzeichnung (z.B. grün). Die Kennzeichnungen können z.B. gleich einer Kennzeichnung von Polen des Magnets (d.h. einem magnetischen Riegelabschnitt) des Riegelelements sein. Dies hat für den Anwender den Vorteil, dass für ihn zu jedem Zeitpunkt (auch unabhängig von einem Feedback des Signalgeberanschlags) unmittelbar ersichtlich ist, in welcher Stellung sich das Riegelelement befindet. D.h., der jeweilige Zustand der Klingenverriegelung kann optisch an den Anwender vermittelt werden. Der Markerabschnitt kann nur die erste und die zweite Kennzeichnung aufweisen oder kann einen schrittweisen oder fließenden Übergang zwischen der ersten und der zweiten Kennzeichnung bzw. zwischen der Offenstellung und der Verriegelungsstellung kennzeichnen.

Weiterhin wäre auch denkbar, dass anhand des Fensters (des optischen Sichtfensters) dargestellt wird, wenn der Anwender zu stark drückt, also ein Druck! eine Flächenpressung im Maulteil/ zwischen den Klemmbacken z.B. größer als 2 N/mm2 beträgt. Beispielsweise kann als Indikator für einen zu starken Druck zwischen den Klemmbacken eine bestimmte Lage des Riegelelements (z.B. einer Verschiebung über die Offenstellung hinaus) oder eine Anzeige/ ein Zustand eines weiteren, hinter dem Fenster im Gehäuse angeordneten Indikatorbauteils dienen, welches den Druck / das Überschreiten eines Grenzdrucks abbildet.

Es ist ferner bevorzugt, wenn der Antriebsmechanismus einen durch einen Anwender betätigbaren Betätigungsschalter zur Betätigung des Gewebetrennelements aufweist. Darüber hinaus kann der Antriebsmechanismus ein Vorspannelement aufweisen, welches das Gewebetrennelement in eine unbetätigte Stellung vorspannt. Dadurch ist es dem Anwender möglich, das Gewebetrennelement nur in einer Richtung (zum Gewebetrennen) triggerartig zu betätigen / auszulösen und im Anschluss kehrt das Gewebetrennelement automatisch zurück in die Ausgangsstellung. Die Anwendung des Instruments ist somit besonders einfach.

Vorteilhafter Weise hat der Antriebsmechanismus eine Antriebs- oder AbtriebsZahnstange und ist das Riegelelement quer zur Zahnstange derart beweglich gelagert, dass es in der Verriegelungsstellung in einen Bewegungspfad der Zahnstange eingreift und sich in der Offenstellung außerhalb des Bewegungspfads der Zahnstange befindet. Dies ist besonders vorteilhaft, da dies eine sehr einfache, bauraumarme und kostengünstige Konstruktion darstellt. Das Riegelelement kann besonders einfach zum Eingriff in den Bewegungspfad der Zahnstange eingreifen. Z.B. kann eine (antreibende) Eingangszahnstange mit dem Betätigungsschalter und eine (abtreibende) Ausgangszahnstange mit dem Gewebetrennelement gekoppelt sein und kann ein Zahnrad diese Zahnstangen koppeln. Alternativ zum Zahnstangenpfad kann das Riegelelement ggf. auch in das Zahnrad eingreifen.

Bevorzugt sind die Zahnstange und das Riegelelement derart ausgebildet und relativ zueinander angeordnet, dass das Riegelelement in der Verriegelungsstellung in Zähne der Zahnstange oder in einen den Zähnen entgegengesetzten Hinterschnitt eingreift. Weiter bevorzugt greift das Riegelelement zumindest in der Verriegelungsstellung U-förmig oder O-förmig um die Zahnstange. In letzterem Fall kann das Riegelelement ferner keilförmig zulaufen. Ferner kann das Riegelelement besonders flexibel, d.h. entlang der gesamten Zahnstange im Instrument angeordnet werden. Die Gesamtkonstruktion ist demgemäß besonders einfach. Zudem ist im Fall des zumindest teilweise U- oder O-förmig ausgebildeten Riegelelements von Vorteil, dass eine Ausführung basierend auf einer (magnetischen oder federnden) Abstoßungskraft zwischen dem Riegelelement und dem Rückstellelement bzw. dem Betätigungsabschnitt einfach auszuführen ist. Alternativ kann das Riegelelement hinter ein Ende einer der Zahnstangen eingreifen.

Ferner ist es von Vorteil, wenn das Rückstellelement ein magnetischer Abschnitt des Antriebsmechanismus sein, wie z.B. eine Führungsschiene- oder platte, in welcher eine der Zahnstangen geführt ist. Dadurch kann der entsprechende Abschnitt eine doppelte Funktion erfüllen und kann auf ein gesondertes Bauteil als Rückstellelement verzichtet werden. Somit wird die Konstruktion weiter vereinfacht und kostengünstiger.

In anderen Worten ausgedrückt, kann die der Erfindung zugrundeliegende Aufgabe zum Beispiel wie folgt gelöst werden.

Ein chirurgisches Instrument hat einen ersten und zweiten Schaft (Instrumentenbranchen), die schwenkbar miteinander verbunden sind, und ein Schneidmesser als ein Beispiel für ein Gewebetrennelement. Um das Schneidmesser zu aktiveren, legt ein Anwender einen Finger auf einen z.B. als Schieber ausgebildeten Cuttertrigger (d.h. einen Betätigungsschalter). Sobald er den Cuttertrigger zu sich zieht, treib dieser eine Zahnstange an, welche über ein Zahnrad eine abtreibende Zahnstange antreibt. Die abtreibende Zahnstange schiebt die Klinge mit sich. Eine Rückstellung dieser Mechanik erfolgt über eine Feder als ein Beispiel für ein Vorspannelement, welche z.B. an der abtreibenden Zahnstange angreift, um diese wieder in die Ausgangslage zu ziehen. Eine Klingenverriegelung zur Verriegelung dieser Mechanik erfolgt magnetisch. Dabei werden z.B. permanente Neodymmagnete mit unterschiedlicher oder gleicher Haltekraft verwendet.

In einem geöffneten Zustand des Instruments wird der Schieber / das Riegelelement durch den Magneten (im Riegelelement oder dem Rückstellelement) in Richtung des Rückstellelements gezogen. Der Schieber blockiert in dieser Position eine Bewegung einer antreibenden / Eingangszahnstange, sodass das Gewebetrennelement nicht betätigt werden kann. Bei einer Annäherung der beiden Instrumentenbranchen wird das Riegelelement immer stärker zu dem magnetischen (ggf. einen Magneten aufweisenden) Betätigungsabschnitt hin angezogen. Dadurch bewegt sich das Riegelelement in Richtung des Betätigungsabschnitts. Wenn das Riegelelement schließlich an einem entsprechenden Anschlag im Gehäuse anschlägt, ist ein Weg für den Antriebsmechanismus / die Klingenmechanik / einen Antriebsstrang zum Antreiben des Gewebetrennelement frei. Das Gewebetrennelement kann also betätigt werden.

Wenn das Instrument sich öffnet bzw. sich die Instrumentenbranchen voneinander entfernen, wird ab einem gewissen Punkt die Anziehung zwischen dem Riegelelement und dem Betätigungselement so schwach, dass die Anziehungskraft zum Rückstellelement stärker wird. Dann bewegt sich der Riegelabschnitt wieder in Richtung des Rückstellelements und damit in die Sperrstellung (Verriegelungsstellung).

### Figurenbeschreibung

Nachfolgend wird die vorliegende Offenbarung anhand bevorzugter Ausführungsformen beschrieben. Diese sind jedoch nur veranschaulichender Natur und sollen den Schutzumfang der vorliegenden Erfindung nicht einschränken. Ferner werden bei der Beschreibung der verschiedenen Ausführungsformen für gleiche Bauteile dieselben Bezugszeichen verwendet, um redundante Beschreibungen derselben zu vermeiden.
Fig. 1 zeigt ein Instrument gemäß einer ersten bevorzugten Ausführungsform der vorliegenden Offenbarung von außen und mit demontiertem Instrumentengehäuse.
Fig. 2 zeigt eine detaillierte Ansicht eines Antriebsmechanismus des Instruments der ersten Ausführungsform.
Fig. 3 und Fig. 4 zeigen einen Verriegelungsmechanismus der ersten Ausführungsform in einer Verriegelungsstellung und einer Offenstellung.
Fig. 5 und Fig. 6 veranschaulichen jeweils eine Modifikation des Verriegelungsmechanismus entsprechend einer zweiten und einer dritten Ausführungsform der vorliegenden Offenbarung.
Fig. 7 und Fig. 8 zeigen eine einen modifizierten Verriegelungsmechanismus nach einer vierten bevorzugten Ausführungsform in einer Verriegelungsstellung und einer Offenstellung.
Fig. 9 und Fig. 10 zeigen eine einen modifizierten Verriegelungsmechanismus nach einer fünften bevorzugten Ausführungsform in einer Verriegelungsstellung und einer Offenstellung.

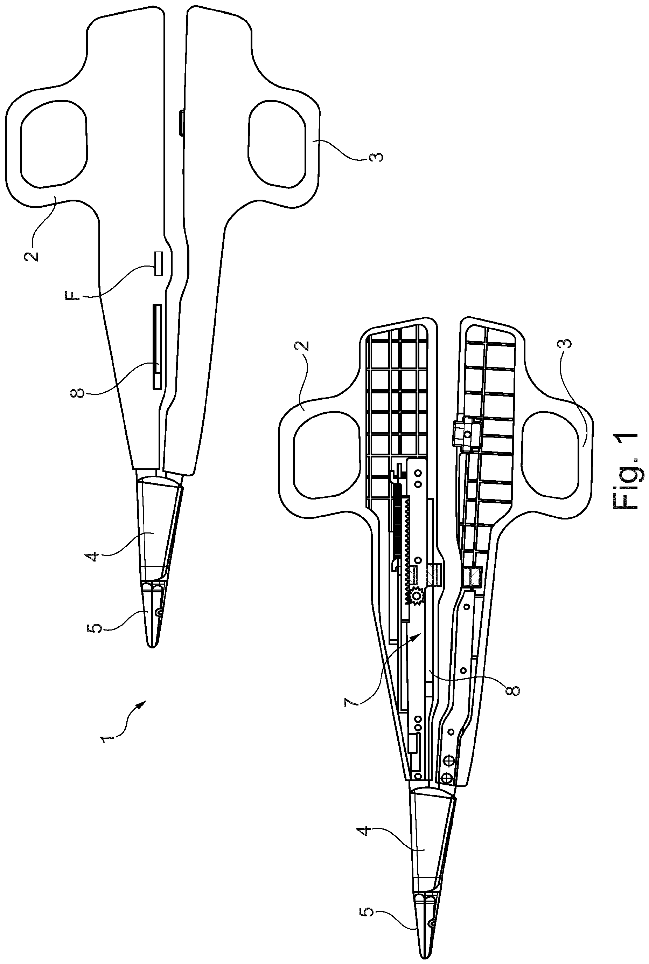

Fig. 1 zeigt ein Instrument 1 gemäß einer ersten bevorzugten Ausführungsform der vorliegenden Offenbarung von außen (oben) und mit demontiertem Instrumentengehäuse (unten). Das Instrument 1 hat eine erste Instrumentenbranche 2 und eine zweite Instrumentenbranche 3, welche an einem Gelenkabschnitt/ Drehgelenk 4 aneinander angelenkt sind. Distal vom Drehgelenk 4 bilden die Instrumentenbranchen 2, 3 Klemmbacken 4 und proximal davon Griffabschnitte. Im Griffabschnitt der zweiten Instrumentenbranche ist ein Antriebsmechanismus 7 angeordnet, welcher über einen Betätigungsschalter 8 durch einen Nutzer betätigbar ist und verriegelbar und entriegelbar ist, wie nachstehend genauer beschrieben ist. Das Gehäuse kann ferner ein Fenster F aufweisen, hinter welchem ein nachstehend genauer beschriebenes Riegelelement 15 angeordnet ist, dessen Oberfläche im Bereich des Fensters F einen Markerabschnitt zur Kennzeichnung eines Zustands/ einer Stellung des Riegelelements 15 ausbilden kann.

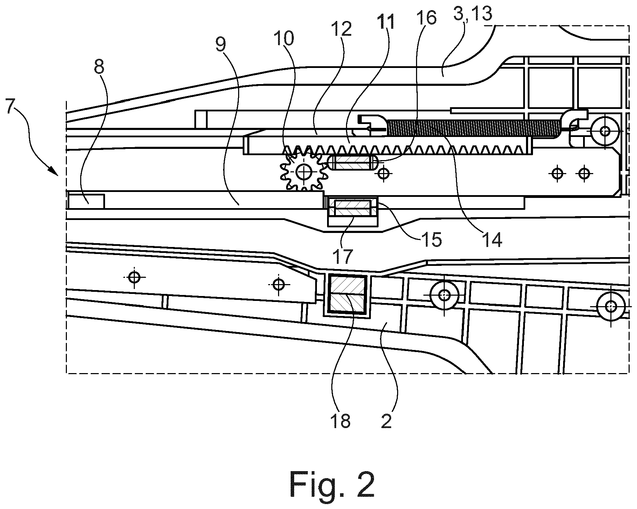

Fig. 2 zeigt eine detaillierte Ansicht eines Antriebsmechanismus 7 des Instruments 1 der ersten Ausführungsform. Die beiden Instrumentenbranchen 2, 3 sind geöffnet, d.h. um das Drehgelenk 4 voneinander weggeschwenkt. Der Betätigungsschalter 8 ist mit einer Antriebs-/Eingangszahnstange 9 verbunden. Die Antriebszahnstange 9 treibt ein Zahnrad 10 an, welches wiederrum eine Abtriebs-/Ausgangszahnstange 11 antreibt. Die Abtriebszahnstange 11 ist mittelbar oder unmittelbar mit einem Gewebetrennelement 12 (hier ein vorschiebbarer Klingenschieber) verbunden. In der vorliegenden Darstellung ist das Gewebetrennelement 12 unbetätigt und verriegelt, d.h. in einer proximalen Position angeordnet. Die Abtriebszahnstange 11 bzw. das Gewebetrennelement 12 ist mit einem Instrumentengehäuse 13 der zweiten Instrumentenbranche 3 über ein Vorspannelement 14 in Form einer Spiralzugfeder verbunden, welche das Gewebetrennelement 12 in eine unbetätigte Ausgangsstellung vorspannt.

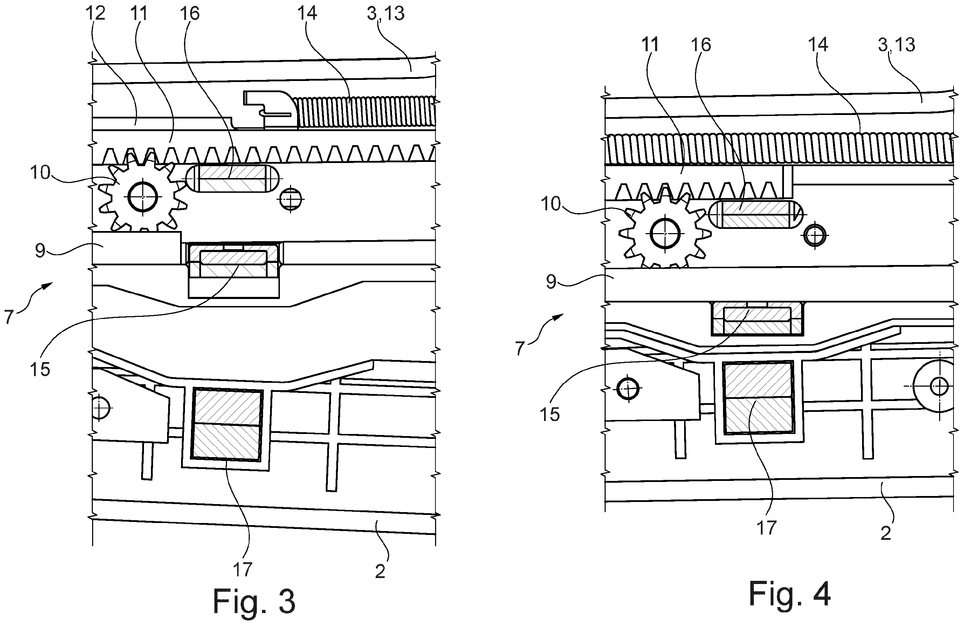

In der vorliegenden Darstellung sowie vergrößert in Fig. 3 ist der Antriebsmechanismus durch einen Verriegelungsmechanismus mit einem Riegelelement 15 blockiert, d.h., das Riegelelement 15 ist in einer Verriegelungsstellung. Das Riegelelement 15 hat einen Magneten der so ausgerichtet ist, dass sein einer Pol der ersten Instrumentenbranche zugewandt ist und der andere Pol davon abgewandt ist. An seinem anderen Pol hat das Riegelelement 15 eine Kappe, welche den Magneten teilweise umschließt und hinter ein proximales Ende der Antriebszahnstange greift. Die Kappe kann als ein Dämpferabschnitt zum Dämpfen eines Anschlages des Riegelelements 15 in der Verriegelungsstellung dienen. Alternativ kann die Kappe als ein Signalgeberabschnitt dienen, welcher z.B. ein besonders lautes Geräusch beim Anschlag des Riegelelements 15 in dessen entsprechender Endposition (d.h. am Signalgeberanschlag) dienen. An einer dem anderen Pol gegenüberliegenden Seite ist im Instrumentengehäuse 13 der zweiten Instrumentenhälfte 3 ein weiterer Magnet als ein Rückstellelement 16 befestigt. Der Magnet des Rückstellelements 16 hat einen Pol, welcher anziehend in Richtung hin zum Magneten des Riegelelement 15 ausgerichtet ist. Das Riegelelement 15 wird durch eine Anziehung zwischen dem Magneten des Riegelelements 15 und dem Rückstellelement 16 in der Verriegelungsstellung gehalten. In einem dem Riegelelement 15 gegenüberliegenden Bereich im Griffabschnitt der ersten Instrumentenbranche 2 ist ein magnetisches Betätigungselement 17 befestigt, welches noch einen weiteren Magneten aufweist, dessen einer Pol zum Riegelelement 15 hin ausgerichtet ist.

In Fig. 4 sind die beiden Instrumentenbranchen 2, 3 geschlossen. Das Betätigungselement 17 ist näher am Magneten des Riegelelements 15 als in der Stellung im Instrument 1 gemäß Fig. 2 und 3. Dadurch ist eine Anziehungskraft zwischen dem Betätigungselement 17 und dem Rückstellelement 16 und dem Riegelelement 15. Dadurch ist das Riegelelement 15 zum Betätigungselement 17 hin verschoben und greift nicht mehr proximal hinter die Antriebszahnstange 9. D.h. das Riegelelement 15 befindet sich in einer Offenstellung. Somit kann die Antriebszahnstange 9, wie hier dargestellt, in Richtung proximal verschoben werden und das Gewebetrennelement 12 betätigen.

Nachfolgend werden weitere Ausführungsformen beschrieben, welche bis auf die nachfolgend erläuterten Unterschiede bevorzugt der ersten Ausführungsform entsprechen.

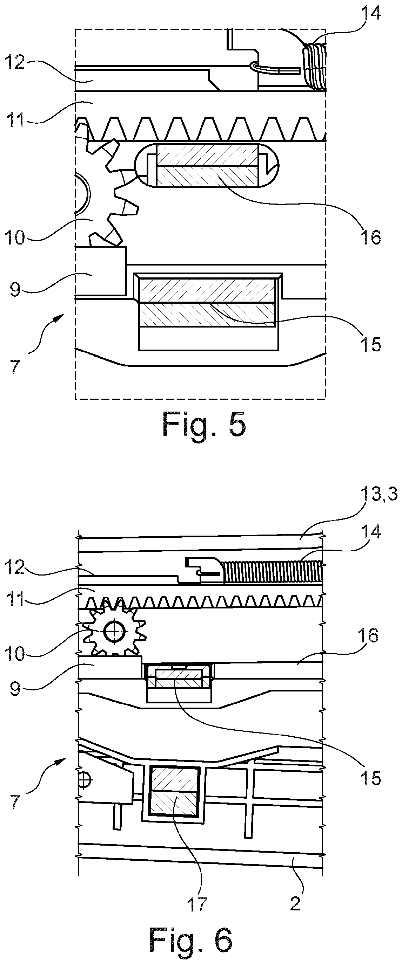

Fig. 5 veranschaulicht eine Modifikation des Verriegelungsmechanismus entsprechend einer zweiten Ausführungsform der vorliegenden Offenbarung. Das Riegelelement 15 hat gemäß dieser Modifikation keine Kappe. Fig. 6 veranschaulicht eine weitere Modifikation des Verriegelungsmechanismus entsprechend einer dritten Ausführungsform der vorliegenden Offenbarung. Darin ist das Rückstellelement 16 durch eine metallische Führungsschiene der Antriebszahnstange 9 ausgebildet. Das Rückstellelement 16 weist somit keinen Magneten auf.

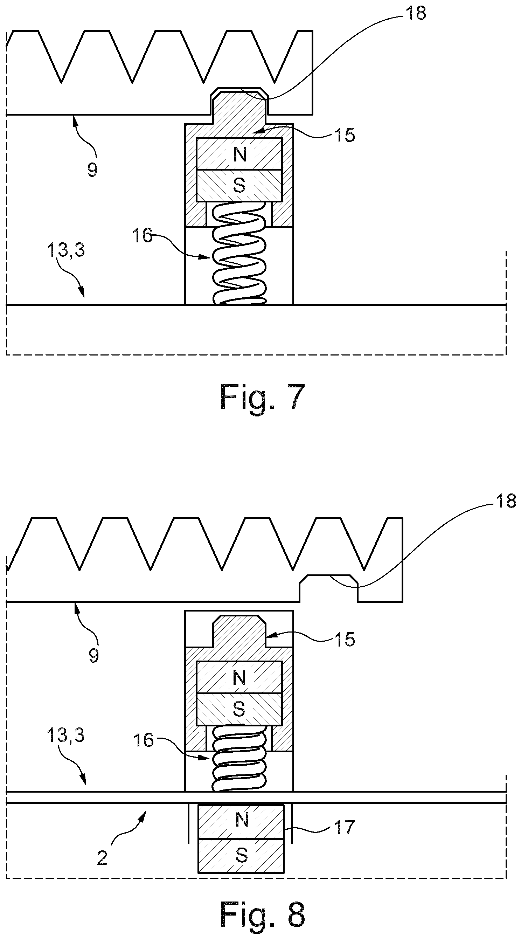

Fig. 7 und Fig. 8 zeigen eine einen modifizierten Verriegelungsmechanismus nach einer vierten bevorzugten Ausführungsform in einer Verriegelungsstellung bzw. in einer Offenstellung. Die Antriebszahnstange 9 hat eine zahnbesetzte Seite und eine zahnfreie Seite. An der Zahnfreien Seite ist ein Hinterschnitt 18 ausgebildet, welcher zum Eingriff mit dem Riegelelement 15 bzw. einer Kappe desselben ausgebildet ist. Das Rückstellelement 16 ist als eine (Spiral-) Feder ausgebildet. Diese stützt sich zwischen dem Riegelelement 15 und dem Instrumentengehäuse 13 der zweiten Instrumentenbranche 3 ab. Nähert sich die erste Instrumentenhälfte 2, wie in Fig. 8 dargestellt, überkommt eine magnetische Anziehungskraft zwischen dem Riegelelement 15 und dem Betätigungsabschnitt 17 eine Federkraft des Rückstellelements 16, sodass das Riegelelement 15 zurückgezogen wird in die Offenstellung.

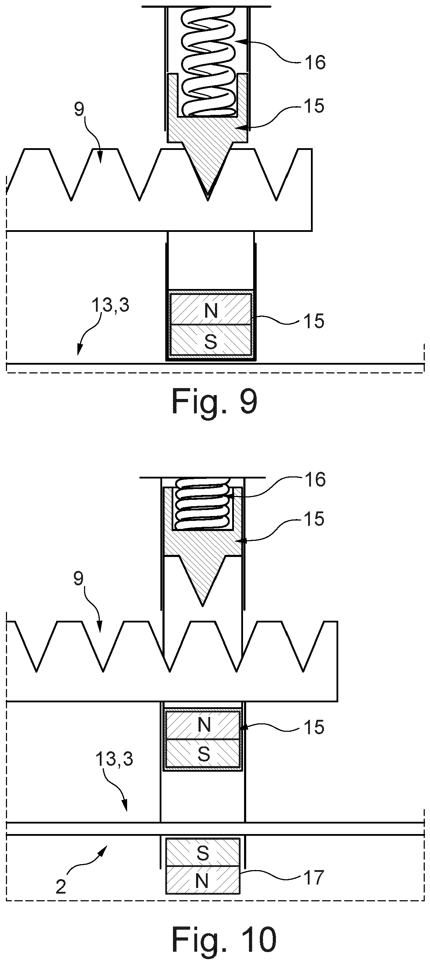

Fig. 9 und Fig. 10 zeigen eine einen modifizierten Verriegelungsmechanismus nach einer fünften bevorzugten Ausführungsform in einer Verriegelungsstellung bzw. einer Offenstellung. Das Riegelelement 15 ist als U- oder O-förmiger Greifer ausgebildet, welcher um die Antriebszahnstange 9 greift. An einer der ersten Instrumentenbranche 2 zugewandten Greiferseite hat das Riegelelement 15 einen Magneten. An seiner der ersten Instrumentenbranche 2 entgegengesetzten Greiferseite hat das Riegelelement einen Eingriffsabschnitt, welcher zum Eingriff in Zähne der Antriebszahnstange 9 ausgebildet ist. Das Rückstellelement 16 als eine (Spiral-) Feder ausgebildet, welche an der der ersten Instrumentenbranche 2 entgegengesetzten Greiferseite gegen das Riegelelement 15 drückt, um dieses, wie in Fig. 9 dargestellt, in die Verriegelungsstellung vorzuspannen. Nähert sich die erste Instrumentenhälfte 2, wie in Fig. 10 dargestellt, überkommt eine magnetische Abstoßungskraft zwischen dem Riegelelement 15 und dem Betätigungsabschnitt 17 eine Federkraft des Rückstellelements 16, sodass das Riegelelement 15 zurückgezogen/ zurückgeschoben wird in die Offenstellung.

### Referenzzeichenliste

- 1: Instrument
- 2: Erste Instrumentenbranche
- 3: Zweite Instrumentenbranche
- 4: Drehgelenk/ Gelenkabschnitt
- 5: Klemmbacken/ Distale Instrumentenspitze
- 7: Antriebsmechanismus
- 8: Betigungsschalter
- 9: Antriebs-/ Eingangszahnstange
- 10: Zahnrad
- 11: Abtriebs-/ Ausgangszahnstange
- 12: Gewebetrennelement/ Klingenschieber
- 13: Gehäuse der zweiten Instrumentenbranche
- 14: Vorspannelement/ Zugfeder
- 15: Magnetisches Riegelelement
- 16: Magnetisches Rückstellelement
- 17: Magnetisches Betätigungselement
- 18: Hinterschnitt
- F: Fenster/ Sichtfenster

## Patentansprüche

1. Chirurgisches Instrument (1) zum Klemmen und Trennen von Patientengewebe, mit einer ersten Instrumentenbranche (2) und einer an der ersten Instrumentenbranche (2) verschwenkbar angelenkten zweiten Instrumentenbranche (3), welche jeweils eine distale Klemmbacke (5) und ein proximales Griffelement ausbilden, wobei
die zweite Instrumentenbranche (3) ein Gewebetrennelement (12) zum Durchtrennen von zwischen den Klemmbacken (5) gehaltenem Patientengewebe und einen Antriebsmechanismus (7) zum Antreiben des Gewebetrennelements (12) aufweist und ein Riegelelement (15) derart lagert, dass es zwischen einer Verriegelungsstellung, in welcher es in den Antriebsmechanismus (7) blockierend eingreift, und einer Offenstellung, in welcher der Antriebsmechanismus (7) durch das Riegelelement (15) unblockiert ist, beweglich ist, **dadurch gekennzeichnet, dass**
das Riegelelement (15) einen magnetischen Riegelabschnitt aufweist und die erste Instrumentenbranche (2) an einem dem Riegelelement (15) gegenüberliegenden Bereich einen magnetischen Betätigungsabschnitt (17) zur Betätigung des Riegelelements (15) aufweist, wobei der magnetische Riegelabschnitt und/oder der magnetische Betätigungsabschnitt (17) einen Magneten aufweist, um bei einer Annäherung der Instrumentenbranchen (2, 3) zueinander das Riegelelement (15) durch eine magnetische Anziehung oder Abstoßung zwischen dem magnetischen Riegelabschnitt und dem ersten magnetischen Betätigungsabschnitt (17) in die Offenstellung zu bewegen und eine Betätigung des Gewebetrennelements (12) freizugeben.

2. Chirurgisches Instrument (1) nach Anspruch 1, wobei die zweite Instrumentenbranche (3) ein Rückstellelement (16) aufweist, welches eine Rückstellkraft an das Riegelelement (15) anlegt, die einer Anziehungskraft oder Abstoßungskraft zwischen dem magnetischen Riegelabschnitt und dem magnetischen Betätigungsabschnitt (17) entgegenwirkt, um eine in Richtung der Verriegelungsstellung wirkende Rückstellkraft auf das Riegelelement (15) auszuüben.

3. Chirurgisches Instrument (1) nach Anspruch 2, wobei der magnetische Betätigungsabschnitt (17), der magnetische Riegelabschnitt und das Rückstellelement (16) derart relativ zueinander ausgelegt und positioniert sind, dass das Riegelelement (15) in einer ersten Stellung der Instrumentenbranchen (2, 3) relativ zueinander in der Verriegelungsstellung gehalten wird und in einer zweiten Stellung der Instrumentenbranchen (2, 3) relativ zueinander in der Offenstellung gehalten wird.

4. Chirurgisches Instrument (1) nach Anspruch 3, wobei die distalen Klemmbacken (5) in der ersten Stellung geöffnet sind und sich in der zweiten Stellung in einer vorbestimmten Klemmstellung befinden.

5. Chirurgisches Instrument (1) nach einem der Ansprüche 2 bis 4, wobei das Rückstellelement (16) eine Feder oder ein magnetisches Element aufweist.

6. Chirurgisches Instrument (1) nach einem der Ansprüche 3 bis 5, wobei der magnetische Betätigungsabschnitt (17), der magnetische Riegelabschnitt und das Rückstellelement (16) derart relativ zueinander ausgelegt und positioniert sind, dass das Riegelelement (15) dann in die Offenstellung wechselt, wenn zwischen den Klemmbacken (5) ein vorbestimmter Druck anliegt.

7. Chirurgisches Instrument (1) nach einem der Ansprüche 1 bis 6, wobei eine Bewegung des Riegelelements (15) durch einen Dämpferanschlag begrenzt ist und am Dämpferanschlag und/oder am Riegelelement (15) auf einer dem Dämpferanschlag zugewandten Seite ein Dämpferabschnitt bereitgestellt ist, um ein Auftreffen des Riegelelements (15) auf den Dämpferanschlag zu dämpfen.

8. Chirurgisches Instrument (1) nach einem der Ansprüche 1 bis 7, wobei eine Bewegung des Riegelelements (15) durch einen Signalgeberanschlag begrenzt ist und der Signalgeberanschlag und/ oder das Riegelelement (15) an einer dem Signalgeberanschlag zugewandten Seite ein Signalgeberabschnitt bereitgestellt ist, der beim Auftreffen des Riegelelements (15) auf den Signalgeberanschlag ein taktiles und/oder hörbares Feedback erzeugt oder verstärkt, um dem Anwender zu signalisieren, dass die Verriegelungsstellung und/ oder die Offenstellung erreicht ist.

9. Chirurgisches Instrument (1) nach einem der Ansprüche 1 bis 8, ferner mit einem Instrumentengehäuse (13), welches im Bereich des Riegelelements (15) ein Fenster (F) aufweist, wobei ein hinter dem Fenster (F) liegender Bereich des Riegelelements (15) einen Markerabschnitt ausbildet, welcher den Verriegelungszustand und/oder den Öffnungszustand kennzeichnet.

10. Chirurgisches Instrument (1) nach einem der Ansprüche 1 bis 9, wobei der Antriebsmechanismus (7) einen durch einen Anwender betätigbaren Betätigungsschalter (8) zur Betätigung des Gewebetrennelements (12) und ein Vorspannelement (14) aufweist, welches das Gewebetrennelement (12) in eine unbetätigte Stellung vorspannt.

11. Chirurgisches Instrument (1) nach einem der Ansprüche 1 bis 10, wobei der Antriebsmechanismus (7) eine Zahnstange (8) aufweist und das Riegelelement (15) quer zur Zahnstange (8) derart beweglich ist, dass es in der Verriegelungsstellung in einen Bewegungspfad der Zahnstange (8) eingreift und sich in der Offenstellung außerhalb des Bewegungspfads der Zahnstange (8) befindet.

12. Chirurgisches Instrument (1) nach Anspruch 11, wobei die Zahnstange (8) und das Riegelelement (15) derart ausgebildet und angeordnet sind, dass das Riegelelement (15) in der Verriegelungsstellung in Zähne der Zahnstange (8) oder in einen den Zähnen entgegengesetzten Hinterschnitt (18) eingreift.

13. Chirurgisches Instrument (1) nach einem der Ansprüche 11 und 12, wobei das Riegelelement (15) zumindest in der Verriegelungsstellung U-förmig um die Zahnstange (8) greift.

## Claims

1. A surgical instrument (1) for clamping and separating patient tissue, having a first instrument branch (2) and a second instrument branch (3) which is pivotably articulated on the first instrument branch (2) and which each form a distal clamping jaw (5) and a proximal gripping element, wherein
the second instrument branch (3) comprises a tissue separating element (12) for separating patient tissue held between the clamping jaws (5) and a drive mechanism (7) for driving the tissue separating element (12) and supports a locking element (15) such that it is movable between a locking position, in which it engages the drive mechanism (7) in a blocking manner, and an opened position, in which the drive mechanism (7) is unblocked by the locking element (15), **characterized in that**
the locking element (15) has a magnetic locking portion and the first instrument branch (2) has a magnetic actuating portion (17) for actuating the locking element (15) at a region opposite the locking element (15), the magnetic locking portion and/or the magnetic actuating portion (17) having a magnet, in order to move the locking element (15) into the opened position by a magnetic attraction or repulsion between the magnetic locking portion and the first magnetic actuating portion (17) when the instrument branches (2, 3) approach each other and to release actuation of the tissue separating element (12).

2. The surgical instrument (1) according to claim 1, wherein the second instrument branch (3) comprises a reset element (16) which applies a restoring force to the locking element (15) which counteracts an attractive force or repulsive force between the magnetic locking portion and the magnetic actuating portion (17) to exert a restoring force on the locking element (15) acting in the direction of the locking position.

3. The surgical instrument (1) according to claim 2, wherein the magnetic actuating portion (17), the magnetic locking portion and the reset element (16) are configured and positioned relative to each other in such a way that the locking element (15) is held in the locking position in a first position of the instrument branches (2, 3) relative to each other and is held in the opened position in a second position of the instrument branches (2, 3) relative to each other.

4. The surgical instrument (1) according to claim 3, wherein the distal clamping jaws (5) are open in the first position and are in a predetermined clamping position in the second position.

5. The surgical instrument (1) according to one of claims 2 to 4, wherein the reset element (16) comprises a spring or a magnetic element.

6. The surgical instrument (1) according to one of claims 3 to 5, wherein the magnetic actuating portion (17), the magnetic locking portion and the reset element (16) are configured and positioned relative to each other such that the locking element (15) changes to the opened position when a predetermined pressure is applied between the clamping jaws (5).

7. The surgical instrument (1) according to one of claims 1 to 6, wherein a movement of the locking element (15) is limited by a damper stop and a damper portion is provided on the damper stop and/or on the locking element (15) on a side facing the damper stop in order to dampen an impact of the locking element (15) on the damper stop.

8. The surgical instrument (1) according to one of claims 1 to 7, wherein a movement of the locking element (15) is limited by a signal transmitter stop and the signal transmitter stop and/or the locking element (15) is provided with a signal transmitter portion on a side facing the signal transmitter stop, which generates or amplifies a tactile and/or audible feedback when the locking element (15) hits the signal transmitter stop, in order to signal to the user that the locking position and/or the opened position has been reached.

9. The surgical instrument (1) according to one of claims 1 to 8, further comprising an instrument housing (13) which has a window (F) in the region of the locking element (15), wherein a region of the locking element (15) lying behind the window (F) forms a marker portion which characterizes the locking state and/or the opening state.

10. The surgical instrument (1) according to one of claims 1 to 9, wherein the drive mechanism (7) comprises an actuating switch (8) actuatable by a user for actuating the tissue separating element (12) and a pre-stressing element (14) which pre-stresses the tissue separating element (12) into an unactuated position.

11. The surgical instrument (1) according to one of claims 1 to 10, wherein the drive mechanism (7) comprises a toothed rack (8) and the locking element (15) is movable transversely to the toothed rack (8) such that in the locking position it engages in a movement path of the toothed rack (8) and in the opened position it is located outside the movement path of the toothed rack (8).

12. The surgical instrument (1) according to claim 11, wherein the toothed rack (8) and the locking element (15) are configured and arranged in such a way that the locking element (15) engages in the locking position in teeth of the toothed rack (8) or in an undercut (18) opposite the teeth.

13. The surgical instrument (1) according to one of claims 11 and 12, wherein the locking element (15) engages around the toothed rack (8) in a U-shape at least in the locking position.

## Revendications

1. Instrument chirurgical (1) pour serrer et séparer du tissu de patient, avec une première branche d'instrument (2) et une seconde branche d'instrument (3) articulée de manière pivotante au niveau de la première branche d'instrument (2), lesquelles forment respectivement une mâchoire de serrage distale (5) et un élément de préhension proximal, dans lequel
la seconde branche d'instrument (3) présente un élément de séparation de tissu (12) pour séparer de tissu de patient maintenu entre les mâchoires de serrage (5) et un mécanisme d'entraînement (7) pour entraîner l'élément de séparation de tissu (12) et supporte un élément de verrou (15) de sorte qu'il soit mobile entre une position de verrouillage, dans laquelle il s'engage dans le mécanisme d'entraînement (7) en le bloquant, et une position d'ouverture, dans laquelle le mécanisme d'entraînement (7) n'est pas bloqué par l'élément de verrou (15), **caractérisé en ce que**
l'élément de verrou (15) présente une section de verrou magnétique et la première branche d'instrument (2) présente, dans une zone opposée à l'élément de verrou (15), une section d'actionnement magnétique (17) pour actionner l'élément de verrou (15), dans lequel la section de verrou magnétique et/ou la section d'actionnement magnétique (17) présente un aimant pour déplacer l'élément de verrou (15) dans la position ouverte par une attraction ou une répulsion magnétique entre la section de verrou magnétique et la première section d'actionnement magnétique (17) lors d'un rapprochement des branches d'instrument (2, 3) l'une de l'autre et pour libérer un actionnement de l'élément de séparation de tissu (12).

2. Instrument chirurgical (1) selon la revendication 1, dans lequel la seconde branche d'instrument (3) présente un élément de rappel (16) qui applique une force de rappel au niveau de l'élément de verrou (15) qui agit contre une force d'attraction ou de répulsion entre la section de verrou magnétique et la section d'actionnement magnétique (17) pour exercer une force de rappel sur l'élément de verrou (15) agissant en direction de la position de verrouillage.

3. Instrument chirurgical (1) selon la revendication 2, dans lequel la section d'actionnement magnétique (17), la section de verrou magnétique et l'élément de rappel (16) sont conçus et positionnés les uns par rapport aux autres de sorte que l'élément de verrou (15) soit maintenu dans la position de verrouillage dans une première position des branches d'instrument (2, 3) les unes par rapport aux autres et soit maintenu dans la position d'ouverture dans une seconde position des branches d'instrument (2, 3) les unes par rapport aux autres.

4. Instrument chirurgical (1) selon la revendication 3, dans lequel les mâchoires de serrage distales (5) sont ouvertes dans la première position et se trouvent dans une position de serrage prédéterminée dans la seconde position.

5. Instrument chirurgical (1) selon l'une quelconque des revendications 2 à 4, dans lequel l'élément de rappel (16) présente un ressort ou un élément magnétique.

6. Instrument chirurgical (1) selon l'une quelconque des revendications 3 à 5, dans lequel la section d'actionnement magnétique (17), la section de verrou magnétique et l'élément de rappel (16) sont conçus et positionnés les uns par rapport aux autres de sorte que l'élément de verrou (15) bascule dans la position ouverte lorsqu'une pression prédéterminée est appliquée entre les mâchoires de serrage (5).

7. Instrument chirurgical (1) selon l'une quelconque des revendications 1 à 6, dans lequel un déplacement de l'élément de verrou (15) est limité par une butée d'amortissement et une section d'amortissement est prévue au niveau de la butée d'amortissement et/ou au niveau de l'élément de verrou (15) sur un côté tourné vers la butée d'amortissement pour amortir un impact de l'élément de verrou (15) sur la butée d'amortissement.

8. Instrument chirurgical (1) selon l'une quelconque des revendications 1 à 7, dans lequel un déplacement de l'élément de verrou (15) est limité par une butée d'émetteur de signal, et la butée d'émetteur de signal et/ou l'élément de verrou (15) est pourvu, au niveau d'un côté tourné vers la butée d'émetteur de signal, d'une section d'émetteur de signal qui, lorsque l'élément de verrou (15) heurte la butée d'émetteur de signal, génère ou amplifie un retour tactile et/ou audible pour signaler à l'utilisateur que la position de verrouillage et/ou la position d'ouverture est atteinte.

9. Instrument chirurgical (1) selon l'une quelconque des revendications 1 à 8, avec en outre un boîtier d'instrument (13) qui présente une fenêtre (F) dans la zone de l'élément de verrou (15), dans lequel une zone de l'élément de verrou (15) située derrière la fenêtre (F) forme une section de marqueur qui caractérise l'état de verrouillage et/ou l'état d'ouverture.

10. Instrument chirurgical (1) selon l'une quelconque des revendications 1 à 9, dans lequel le mécanisme d'entraînement (7) présente un commutateur d'actionnement (8) pouvant être actionné par un utilisateur pour actionner l'élément de séparation de tissu (12) et un élément de précontrainte (14) qui précontraint l'élément de séparation de tissu (12) dans une position non actionnée.

11. Instrument chirurgical (1) selon l'une quelconque des revendications 1 à 10, dans lequel le mécanisme d'entraînement (7) présente une crémaillère (8) et l'élément de verrou (15) est mobile transversalement à la crémaillère (8) de sorte que, dans la position de verrouillage, il s'engage dans un chemin de déplacement de la crémaillère (8) et, dans la position d'ouverture, il se trouve hors du chemin de déplacement de la crémaillère (8).

12. Instrument chirurgical (1) selon la revendication 11, dans lequel la crémaillère (8) et l'élément de verrou (15) sont conçus et disposés de sorte que, dans la position de verrouillage, l'élément de verrou (15) s'engage dans des dents de la crémaillère (8) ou dans une contre-dépouille (18) opposée aux dents.

13. Instrument chirurgical (1) selon l'une quelconque des revendications 11 et 12, dans lequel l'élément de verrou (15) s'engage en forme de U autour de la crémaillère (8) au moins dans la position de verrouillage.
